(19) Europäisches Patentamt
European Patent Office
Office européen des brevets

(11) Publication number: **0 075 868**
**A2**

# (12) EUROPEAN PATENT APPLICATION

(21) Application number: 82108806.9

(22) Date of filing: 23.09.82

(51) Int. Cl.³: C 07 C 91/16
C 07 C 93/14
//C07C103/38

(30) Priority: 24.09.81 JP 151786/81

(43) Date of publication of application:
06.04.83 Bulletin 83/14

(84) Designated Contracting States:
BE CH DE FR GB IT LI

(71) Applicant: SUMITOMO CHEMICAL COMPANY, LIMITED
15 Kitahama 5-chome Higashi-ku
Osaka-shi Osaka-fu(JP)

(72) Inventor: Aratani, Tadatoshi
11-6, Tsutoinari-cho
Nishinomiya Hyogo(JP)

(72) Inventor: Hazama, Motoo
1-3, Wakayamadai Shimamoto-cho
Mishima-gun Osaka(JP)

(72) Inventor: Yoneyoshi, Yukio
3-30-16, Hiyoshidai
Otsu Shiga(JP)

(72) Inventor: Suzukamo, Gohfu
2-1-216, Kuwata-cho Ibaraki
Osaka(JP)

(74) Representative: Vossius Vossius Tauchner Heunemann Rauh
Siebertstrasse 4 P.O. Box 86 07 67
D-8000 München 86(DE)

## (54) Optically active amino alcohols and their production.

(57) An optically active amino alcohol of the formula:

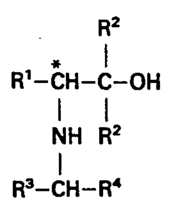

(I)

wherein $R^1$ is a lower alkyl group, a benzyl group or a phenyl group, $R^2$ is a benzyl group, a phenyl group or a phenyl group substituted with at least one of lower alkyl and lower alkoxy, $R^3$ and $R^4$ are each a hydrogen atom, a lower alkyl group or a phenyl group and the carbon atom accompanied by an asterisk (*) indicates an asymmetric carbon atom, which is useful as a chiral reagent for asymmtric synthesis of intermediates in the production of biotin.

EP 0 075 868 A2

Croydon Printing Company Ltd.

OPTICALLY ACTIVE AMINO ALCOHOLS AND THEIR PRODUCTION

The present invention relates to optically active amino alcohols and their production.

This invention provides optically active amino alcohols of the formula:

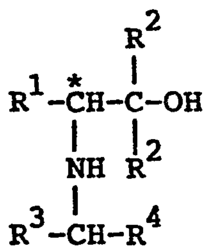

(I)

wherein $R^1$ is a lower alkyl group, a benzyl group or a phenyl group, $R^2$ is a benzyl group, a phenyl group or a phenyl group substituted with at least one of lower alkyl and lower alkoxy, $R^3$ and $R^4$ are each a hydrogen atom, a lower alkyl group or a phenyl group and the carbon atom accompanied by an asterisk (*) indicates an asymmetric carbon atom.

In the above significances, a group with the term "lower" is intended to mean any atomic group having not more than 4 carbon atoms. Therefore, examples of lower alkyl are methyl, ethyl, n-propyl, isopropyl, n-butyl, isobutyl, t-butyl, etc., and examples of lower alkoxy are methoxy, ethoxy, n-propoxy, isopropoxy, n-butoxy, isobutoxy, etc. Examples of the phenyl group substituted with at least one of lower alkyl and lower alkoxy are 2-methoxyphenyl, 2-isopropoxyphenyl, 2-isopropoxy-5-methylphenyl, etc.

The optically active amino alcohols (I) are novel and useful as reagents for asymmetric synthesis. For instance, their use as the chiral reagents for asymmetric synthesis of intermediates in the synthesis of biotin accomplishes a high asymmetric yield (cf. EP 0044158A, which corresponds to Japanese Patent Publication (unexamined) No. 21374/1982).

According to the present invention, the optically active amino alcohol (I), i.e. the secondary amino tertiary alcohol, can be produced from the corresponding primary amino tertiary alcohol by (A) converting the primary amino group of the latter into a Schiff base, followed by reduction, or by (B) acylating the primary amino group of the latter, followed by reduction of the resulting acylated amino group as shown below:

(A) (B)

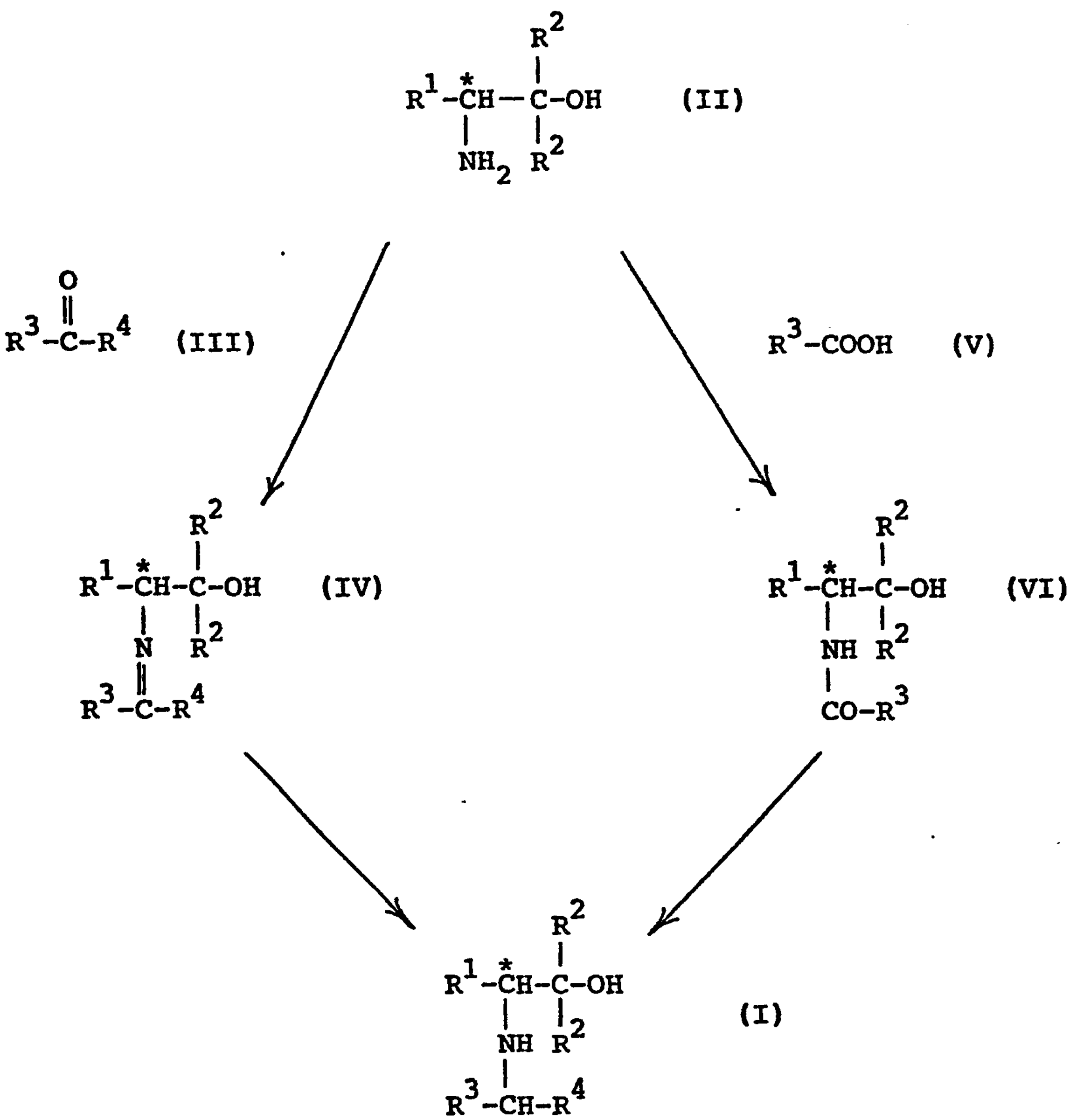

(II)

(III)

(V)

(IV)

(VI)

(I)

wherein $R^1$, $R^2$, $R^3$ and $R^4$ are each as defined above.

The starting material is the optically active amino alcohol (II), i.e. the primary amino tertiary alcohol. It may be in the form of (R)-isomer or (S)-isomer. It is generally known and can be produced by conventional procedures (cf. J.Chem.Soc., 127, 291 (1925) and 128, 785 (1926);

Yakugaku Zasshi (Bull.Pharm.Soc.Japan), 48, 46 (1928); Japanese Patent Publication (examined) No. 13133/1976). Specific examples are 2-amino-1,1-diphenyl-1-propanol, 2-amino-1,1-dibenzyl-1-propanol, 2-amino-1,1-diphenyl-4-methyl-1-pentanol, 2-amino-1,1-dibenzyl-4-methyl-1-pentanol, 2-amino-1,1,2-triphenylethanol, 2-amino-1,1,3-triphenyl-1-propanol, 2-amino-1,1-di(2-methoxyphenyl)-1-propanol, 2-amino-1,1-di(2-isopropoxyphenyl)-3-phenyl-1-propanol, 2-amino-1,1-di(2-isopropoxy-5-methylphenyl)-3-phenyl-1-propanol, etc.

According to the procedure (A), the optically active aminoalcohol (II) is reacted with the carbonyl compound (III), and the resulting Schiff base (IV) is reduced to give the objective optically active amino alcohol (I).

Specific examples of the carbonyl compound (III) are formaldehyde, acetaldehyde, propionaldehyde, butyraldehyde, benzaldehyde, acetone, methyl ethyl ketone, acetophenone, etc.

The reaction between the optically active amino alcohol (II) and the carbonyl compound (III) is usually carried out in an inert solvent such as an aromatic hydrocarbon (e.g. benzene, toluene) or a lower alkanol (e.g. methanol, ethanol) at a temperature of about 20°C to the boiling point of the solvent, preferably with removal of water, for instance, by azeotropic distillation. The molar ratio of the optically active amino alcohol (II) and the

carbonyl compound (III) may be normally from 0.8 - 1.2 : 1. The yield of the Schiff base (IV) is almost quantitative. The Schiff base (IV) may be subjected to reduction in the subsequent step with or without its isolation from the reaction mixture.

The reduction of the Schiff base (IV) may be accomplished by the use of a metal hydride or by catalytic hydrogenation. In the former case, the Schiff base (IV) may be treated with a metal hydride (e.g. lithium aluminum hydride, sodium aluminum hydride, sodium di(methoxyethoxy)-aluminum hydride, sodium borohydride, diborane), usually in an inert solvent such as an ether (e.g. ether, tetrahydrofuran) or a lower alkanol (e.g. methanol, ethanol) at a temperature from 0°C to the boiling point of the solvent. The molar ratio of the Schiff base (IV) and the metal hydride is normally 1 : 0.5 - 20.

In the latter case, the Schiff base (IV) may be treated with hydrogen in the presence of a catalyst such as palladium, palladium oxide, platinum, platinum oxide, nickel or nickel oxide, when desired, deposited on a carrier such as activated charcoal or alumina, usually in an inert solvent such as a lower alkanol (e.g. methanol, ethanol) at a temperature of 0°C to the boiling point of the solvent, particularly around room temperature under a hydrogen pressure of 1 to 100 atm. The amount of the catalyst may be normally from 0.1 to 10 % by weight on the basis of the weight of the Schiff base (IV).

According to the procedure (B), the optically active amino alcohol (II) is reacted with the carboxylic acid (V) or its reactive derivative and the resulting N-acylamino alcohol (VI) is reduced to give the objective optically active amino alcohol (I).

As the carboxylic acid (V), there may be exemplified formic acid, acetic acid, propionic acid, butyric acid, benzoic acid, etc. Examples of its reactive derivative are anhydrides (e.g. mixed acid anhydride of formic acid and acetic acid, acetic anhydride, propionic anhydride, benzoic anhydride), halides (e.g. acetyl chloride, acetyl bromide, butyryl chloride, benzoyl chloride), etc.

The reaction between the otically active amino alcohol (II) and the carboxylic acid (V) or its reactive derivative may be carried out, for instance, in an inert solvent such as an ether (e.g. diethyl ether, tetrahydrofuran), an aromatic hydrocarbon (e.g. benzene, toluene) or a halogenated hydrocarbon (e.g. chloroform, methylene chloride), preferably in the presence of a base such as a tertiary amine (e.g. pyridine, triethylamine, tributylamine) at a temperature of -78°C to the boiling point of the solvent, particularly of -20 to 40°C. When the carboxylic acid (V) or its reactive derivative is an oil, it may be used in an excessive amount so as to serve by itself as the reaction medium. In such case, the said solvent is not necessarily required to use. The molar ratio of the optically active amino alcohol (II) and the carboxylic acid (V) or its

reactive derivative is normally 1 : 1 - 20. The yield of the N-acylamino alcohol (VI) is almost quantitative.

The reduction of the N-acylamino alcohol (VI) may be accomplished by the use of a metal hydride. Thus, the N-acylamino alcohol (VI) is treated with a metal hydride (e.g. lithium aluminum hydride, sodium aluminum hydride, sodium di(methoxyethoxy)aluminum hydride, aluminum hydride, sodium borohydride mixed with aluminum chloride, diborane), usually in an inert solvent such as an ether (e.g. diethyl ether, tetrahydrofuran, dioxane, dimethoxyethane, diglyme) or an aromatic hydrocarbon (e.g. benzene, toluene) at a temperature of 0°C to the boiling point of the solvent. The molar ratio of the N-acylamino alcohol (VI) and the metal hydride is normally 1 : 0.5 - 20.

The separation and purification of the thus prepared optically active amino alcohol (I) from the reaction mixture may be achieved by a per se conventional procedure such as extraction of its mineral acid salt (e.g. hydrochloride, sulfate) with water, recrystallization from an appropriate solvent or column chromatography on a suitable adsorbent.

Specific examples of the optically active amino alcohol (I) are N-methyl-2-amino-1,1-diphenyl-1-propanol, N-ethyl-2-amino-1,1-diphenyl-1-propanol, N-isopropyl-2-amino-1,1-diphenyl-1-propanol, N-benzyl-2-amino-1,1-diphenyl-1-propanol, N-benzyl-2-amino-1,1-diphenyl-4-methyl-1-pentanol, N-methyl-2-amino-1,1-dibenzyl-1-propanol, N-

methyl-2-amino-1,1-diphenyl-4-methyl-1-pentanol, N-isopropyl-2-amino-1,1-diphenyl-4-methyl-1-pentanol, N-methyl-2-amino-1,1-dibenzyl-4-methyl-1-pentanol, N-methyl-2-amino-1,1,2-triphenylethanol, N-methyl-2-amino-1,1,3-triphenyl-1-propanol, N-methyl-2-amino-1,1-di(2-methoxyphenyl)-1-propanol, N-methyl-2-amino-1,1-di(2-isopropoxyphenyl)-3-phenyl-1-propanol, N-methyl-2-amino-1,1-di(2-isopropxoy-5-methylphenyl)-3-phenyl-1-propanol, etc.

Practical and presently preferred embodiments of the invention are illustratively shown in the following examples wherein % and parts are by weight unless otherwise indicated.

Example 1

(1) Preparation of (S)-N-formyl-2-amino-1,1-diphenyl-1-propanol:-

To a solution of mixed acid anhydride of formic acid with acetic acid, which was prepared by heating a mixture of acetic anhydride (30 ml) and formic acid (13 ml) at 50 to 60°C for 2 hours, (S)-2-amino-1,1-diphenyl-1-propanol (45.4 g; 0.2 mol) was added at room temperature, and the resulting mixture was stirred at room temperature for 24 hours. The reaction mixture was admixed with chloroform (200 ml) and washed successively with water (100 ml) twice, saturated sodium bicarbonate solution (100 ml) twice, N hydrochloric acid (100 ml) once and water (100 ml) once. The washed chloroform solution was dried over anhydrous sodium sulfate and concentrated to give (S)-N-formyl-2-amino-1,1-diphenyl-1-propanol (46.6 g) as white crystals. Yield, 91.4 %.

M.P., 162.1°C. $[\alpha]_D$ -98.1° (c=100, $CHCl_3$). IR $\nu_{NH}$ 3330 $cm^{-1}$. $\nu_{co}$ 1660, 1640 $cm^{-1}$.

(2) Preparation of (S)-N-methyl-2-amino-1,1-diphenyl-1-propanol:-

A solution of (S)-N-formyl-2-amino-1,1-diphenyl-1-propanol (40.0 g; 0.156 mol) obtained in (1) in tetrahydrofuran (100 ml) was dropwise added to a suspension of lithium aluminum hydride (11.78 g; 0.31 mol) in tetrahydrofuran (150 ml) in 1 hour at room temperature in nitrogen atmosphere, whereby heat was generated to elevate the inner temperature up to 60°C. The reaction mixture was refluxed for 5 hours. After cooling with ice, water (20 ml) and then 12 % sodium hydroxide solution (40 ml) were added thereto to decompose lithium aluminum hydride. The resulting mixture was admixed with celite (10 g) and filtered. The residue was washed with tetrahydrofuran (100 ml). The filtrate and the washing solution were combined together, dried over anhydrous sodium sulfate and concentrated. The residue was purified by colum chromatography on silica gel to give (S)-N-methyl-2-amino-1,1-diphenyl-1-propanol (29.21 g) as an oily product. Yield, 88.4 %.

$[\alpha]_D$ -64.1° (c=1.02, $CHCl_3$). NMR (τ) 2.3 - 2.93 (m, 10H, ØH), 6.37 (q, 1H, CH), 8.70 (s, 3H, N-$CH_3$), 9.05 (d, 3H, $CH_3$). Elemental analysis ($C_{16}H_{19}NO$): Calcd.: C, 79.63 %; H, 7.94 %; N, 5.80 %. Found: C, 80.01 %; H,

8.03 %; N, 5.62 %.

Examples 2 to 9

In the same manner as in Example 1, the starting amino alcohol (II) was converted into the corresponding N-methyl compound (I) through the corresponding N-formyl compound (VI).

The results are shown in Table 1.

Table 1

| Example No. | Amino alcohol | N-Formyl compound | | | N-Methyl compound | | |
|---|---|---|---|---|---|---|---|
| | | Yield (%) | M.P. (°C) | $[\alpha]_D$ (°) *) | Yield (%) | M.P. (°C) | $[\alpha]_D$ (°) *) |
| 2 | (S)-2-Amino-1,1-dibenzyl-1-propanol | 85.2 | 99.9 | -37.5 | 78.1 | 69.8 | +43.6 |
| 3 | (S)-2-Amino-1,1-diphenyl-4-methyl-1-pentanol | 100 | 52.1 | -60.1 | 92.5 | Oil | -32.8 |
| 4 | (S)-2-Amino-1,1-dibenzyl-4-methyl-1-pentanol | 91.0 | Oil | -24.5 | 88.0 | Oil | +25.9 |
| 5 | (R)-2-Amino-1,1,2-triphenylethanol | 100 | - | - | 70.4 | 117.8 | +178.6 |
| 6 | (R)-2-Amino-1,1,3-triphenyl-1-propanol | 96.4 | 147.1 | +72.2 | 87.9 | 85.1 | -11.1 |
| 7 | (S)-2-Amino-1,1-di(2-methoxyphenyl)-1-propanol | 90 | 160.5 | -158.8 | 25.9 | 97.5 | -59.4 |
| 8 | (R)-2-Amino-1,1-di(2-isopropoxyphenyl)-3-phenyl-1-propanol | 96.9 | 41.9 | +155.3 | 69.8 | Oil | +30.1 |
| 9 | (R)-2-Amino-1,1-di(2-isopropoxy-5-methylphenyl)-3-phenyl-1-propanol | 92.2 | 132.1 | +195.5 | 80.1 | Oil | +53.2 |

Note: *) (c=1, $CHCl_3$)

Example 10

Preparation of (S)-N-ethyl-2-amino-1,1-diphenyl-1-propanol:-

A mixture of (S)-2-amino-1,1-diphenyl-1-propanol (11.35 g; 50 mmol) and acetaldehyde (2.64 g; 60 mmol) in ethanol (100 ml) was stirred at room temperature for 2 hours. Analysis of the reaction mixture on thin layer chromatography showed the disappearance of the starting amino alcohol and the appearance of the Schiff base.

Sodium borohydride (3.8 g; 100 mmol) was added to the reaction mixture, and the resulting mixture was refluxed for 1.5 hours. The solvent was distilled and the residue was extracted with chloroform. The chloforom extract was washed with water, dried over anhydrous sodium sulfate and concentrated. Purification by column chromatography on silica gel gave (S)-N-ethyl-2-amino-1,1-diphenyl-1-propanol (12.80 g) as an oil. Yield, 100 %.

$[\alpha]_D$ -50.3° (c=1.05, $CHCl_3$). NMR (τ) 2.4-3.0 (m, 10H, $-C_6H_6$), 6.3 (q, 1H, CH), 7.5 (m, 2H, $CH_2$), 9.05 (t, 3H, $CH_2$), 9.07 (d, 3H, $CH_3$).

Hydrogenation with 5 % palladium carbon under the atmospheric pressure in place of reduction with sodium borohydride gave the same product as above.

Example 11

Preparation of (S)-N-benzyl-2-amino-1,1-diethyl-1-propanol:-

A mixture of (S)-2-amino-1,1-diphenyl-1-propanol

(9.09 g), benzaldehyde (4.4 g) and ethanol (75 ml) was stirred while refluxing for 2.5 hours. The reaction mixture was concentrated under reduced pressure. Recrystallization of the residue from isopropanol (60 ml) gave the corresponding N-benzylidene product (9.15 g) as colorless crystals. Yield, 72.6 %.

IR (1640 $cm^{-1}$). NMR ($\tau$) 1.65 and 7.75.

A mixture of the N-benzylidene product (7.3 g), sodium borohydride (0.73 g) and ethanol (40 ml) was stirred at 60°C for 8 hours. The reaction mixture was allowed to stand at room temperature overnight. The precipitated crystals (5.8 g) were collected by filtration and recrystallized from ethanol (35 ml) to give (S)-N-benzyl-2-amino-1,1-diethyl-1-propanol (3.80 g) as colorless crystals. Yield, 51.7 %.

$[\alpha]_D$ -37.1° (c=1.0, chloroform).

Example 12

Preparation of (S)-N-benzyl-2-amino-1,1-diphenyl-4-methyl-1-pentanol:-

In the same manner as above, (S)-2-amino-1,1-diphenyl-4-methyl-1-pentanol was reacted with benzaldehyde to give the corresponding N-benzylidene product. Yield, 77.2 %.

In the same manner as above, the N-benzylidene product was reduced with sodium borohydride to give (S)-N-benzyl-2-amino-1,1-diphenyl-4-methyl-1-pentanol. Yield, 98 %.

M.P., 64 - 70°C. $[\alpha]_D$ -22.1° (c=1.0, chloforom).

Example 13

Preparation of (S)-N-isopropyl-2-amino-1,1-diphenyl-1-propanol:-

Into an autoclave, platinum oxide previously absorbed with hydrogen (Adams catalyst) (0.15 g), (S)-amino-1,1-diphenyl-1-propanol (10.0 g; 44 mmol), acetone (5.0 g; 86 mmol), ethanol (10 ml) and toluene (20 ml) were charged, and the atmosphere was replaced by hydrogen to make a pressure of 6.7 to 6.9 atm. The mixture was stirred at room temperature vigorously. Absorption of hydrogen stopped after 3 days. After removal of the catalyst from the reaction mixture by filtration, the filtrate was concentrated under reduced pressure. The residue was recrystallized from methanol to give (S)-N-isopropyl-2-amino-1,1-diphenyl-1-propanol (11.7 g) as colorless crystals. Yield, 99 %.

M.P., 93.6°C. $[\alpha]_D$ -45.0° (c=1.02, chloforom).

Example 14

Preparation of (S)-N-isopropyl-2-amino-1,1-diphenyl-4-methyl-1-pentanol:-

In the same manner as in Example 1, (S)-2-amino-1,1-diphenyl-4-methyl-1-pentanol was converted into (S)-N-isopropyl-2-amino-1,1-diphenyl-4-methyl-1-pentanol as colorless crystals. Yield, 87 %.

M.P., 101.7°C. $[\alpha]_D$ -28.3° (c=1.02, chloroform).

What is claimed is:

1. An optically active amino alcohol of the formula:

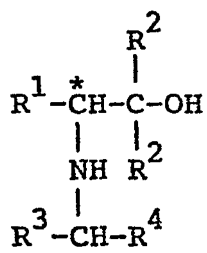

(I)

wherein $R^1$ is a lower alkyl group, a benzyl group or a phenyl group, $R^2$ is a benzyl group, a phenyl group or a phenyl group substituted with at least one of lower alkyl and lower alkoxy, $R^3$ and $R^4$ are each a hydrogen atom, a lower alkyl group or a phenyl group and the carbon atom accompanied by an asterisk (*) indicates an asymmetric carbon atom.

2. A process for preparing the optically active amino alcohol according to claim 1, which comprises reacting an optically active amino alcohol of the formula:

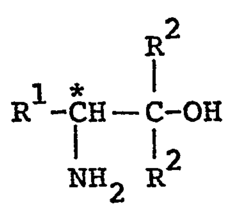

(II)

wherein $R^1$ and $R^2$ are each as defined in claim 1 with a carbonyl compound of the formula:

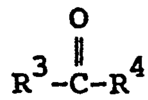 (III)

wherein $R^3$ and $R^4$ are each as defined in claim 1, followed by reduction of the resulting Schiff base of the formula:

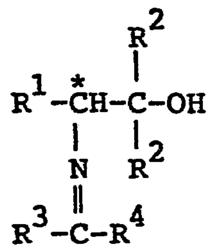 (IV)

wherein $R^1$, $R^2$, $R^3$ and $R^4$ are each as defined in claim 1.

3. A process for preparing the optically active amino alcohol according to claim 1, which comprises reacting an optically active amino alcohol of the formula:

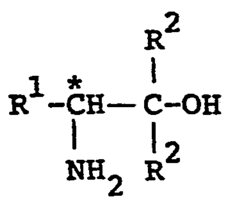 (II)

wherein $R^1$ and $R^2$ are each as defined in claim 1 with a carboxylic acid of the formula:

$R^3$-COOH (V)

wherein $R^3$ is as defined in claim 1 or its reactive derivative, followed by reduction of the resulting N-acylamino alcohol of the formula:

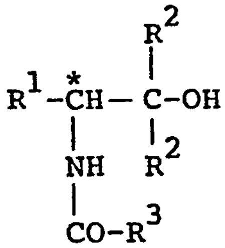

(VI)

wherein $R^1$, $R^2$ and $R^3$ are each as defined in claim 1.